# EUROPEAN PATENT APPLICATION

(11) **EP 2 143 730 A2**
(43) Date of publication of application: **13.01.2010**
(21) Application number: 09174651.1
(22) Date of filing: 06.09.2002
(51) Int. Cl.: C07K 14/47, A61K 38/02, A61K 38/16, A61K 39/00

(54) **Modified peptides and their use for the treatment of autoimmune diseases**

(30) Priority: 06.09.2001 US 317737 P
(62) Divisional of application: 02797690.1
(71) Applicant: Centre National de la Recherche Scientifique (CNRS), 75794 Paris (FR)
(72) Inventor: Muller, Sylviane, 67000, STRASBOURG (FR); Monneaux, Fanny, 67610, LA WANTZENAU (FR); Briand, Jean-Paul, 67000, STRASBOURG (FR); Guichard, Gilles, 33170, GRADIGNAN (FR); Guillet, Jean-Gérard (deceased), 75009, FRANCE (FR)
(74) Representative: Mouget-Goniot, Claire

(57) **Abstract**

A modified peptide in which at least one of the amino acid residues constituting the peptide has been chemically modified, by phosphorylation, acetylation or methylation, in particular peptide of sequence RIHMVYSKRSGKPRGYAFIEY [SEQ ID NO: 1].

SEQUENCE LISTING

<110>  CENTRE NATIONAL DE LA RECHERCHE SCIENTIFIQUE
       MULLER, Sylviane
       MONNIAUX, Fanny
       BRIAND, Jean-Paul
       GUICHARD, Gilles
       GUILLET, Jean-Gérard

<120>  Modified peptides and their use for the treatment of autoimmune
       disease

<130>  EPD 09 MZ ZIM PUSU

<150>  US 60/317,737
<151>  2001-09-06

<160>  1

<170>  PatentIn version 3.5

<210>  1
<211>  21
<212>  PRT
<213>  Homo sapiens

<400>  1

Arg Ile His Met Val Tyr Ser Lys Arg Ser Gly Lys Pro Arg Gly Tyr
1               5                   10                  15

Ala Phe Ile Glu Tyr
            20

## Description

### FIELD OF THE INVENTION

The subject matter of the present invention is directed toward compositions and methods for the treatment of autoimmune diseases, specifically those diseases where it can be shown that the autoimmune process contributes to the pathogenesis of the disease.

### BACKGROUND OF THE INVENTION

### Autoimmune Diseases

These disorders may be looked upon as forming a spectrum. At one end, we have "organ-specific diseases" with organ-specific auto-antibodies.
Hashimoto's disease of the thyroid is an example. In this disorder, there is a specific lesion in the thyroid involving infiltration by mononuclear cells (lymphocytes, histiocytes and plasma cells), destruction of follicular cells and germinal center formation, accompanied by the production of circulating antibodies with absolute specificity for certain thyroid constituents. Towards the center of the spectrum are those disorders where the lesion tends to be localized to a single organ but the antibodies are non-organ-specific. A typical example would be primary biliary cirrhosis where the small bile ductule is the main target of inflammatory cell infiltration but the serum antibodies present-mainly mitochondrial-are not liver specific.

### Systemic Lupus Erythematosus (SLE)

At the other end of the spectrum are the "non-organ-specific" or "systemic autoimmune diseases," broadly belonging to the class of rheumatological disorders, exemplified by systemic lupus erythematosus (SLE), where both lesions and auto-antibodies are not confined to any one organ.
Pathological changes are widespread and are primarily lesions of connective tissue with fibrinoid necrosis. They are seen in the skin (the "lupus" butterfly rash on the face is characteristic), kidney glomeruli, joints, serous membranes and blood vessels. In addition, the formed elements of the blood are often affected. A bizarre collection of auto-antibodies are found, some of which react with the DNA and other nuclear constituents, of all cells in the body.

SLE predominantly affects women and is more common in blacks.
Although survival rates have improved, over one half of patients with SLE have permanent damage in one or more organ systems. Arthritis and cutaneous manifestations are most common, but renal, hematolgic and neurologic manifestations contribute largely to morbidity and mortality.

The pathophysiology of SLE is not completely understood. The production of abnormal antibodies by B cells remains the hallmark sign of SLE.
Some of the auto-antibodies, such as anti-double-stranded DNA and anti-Smith, are very specific for SLE. Others, including anti-RNP, anti Rho and anto-La, are also present in other autoimmune diseases. Whether the B cells themselves are intrinsically abnormal is a subject of current research. One of the underlying defects in SLE may center on apoptosis, or programmed cell death. In patients with SLE, cellular antigens exposed during apoptosis incite an immune response.

Examples of other major diseases considered to be associated with autoimmunity include: primary myxedema, pernicious anemia, Addison's disease, myasthenia gravis, juvenile diabetes, idiopathic thrombocytopenic purpura, ulcerative colitis, multiple sclerosis, rheumatoid arthritis, and scleroderma.

It has been proposed that a pre-existing network linked to epitopes of specific antigens exists and that malfunctioning of this network.

The prior art discloses peptides or proteins having posttranslational modifications such as phosphorylation (DD 205 340 A), acetylation (DD 240 831 A) or acetylation and succinylation (US-A-5 527 688).
The prior art also discloses the use of modified polypeptides for treating SLE. International application WO 00/20583 A teaches that peptides analogous to the amino acid residues 282-328 of human U1 small nuclear ribonucleoprotein 70Kd (snRNP70), can be used for the treatment of autoimmune diseases, such as SLE.
US-A-5 561 222 discloses peptides derived from snRNP70 that are autoantigenic for patients with SLE.
US-A-5 541 291 teaches snRNP70 is useful for the diagnosis and the treatment of autoimmune diseases, including SLE.
The international application WO 95/25124 A teaches that an amino acid sequence derived from 70k snRNP is an autoantigen, and can be used in a pharmaceutical composition for treating autoimmune diseases such as SLE.
Thiessen H et al. (EMBO J. 1986, vol. 5, no. 12, pages 3209-3218) discloses that antigenic peptides of snRNP70 are recognized by antibodies, said antibodies being found in patients with SLE.
The prior art discloses U1 small nuclear ribonucleoprotein 70Kd containing an immunogenic sequence RIHMVYSKRSGKPRGYAFIEY.
However, none of these documents suggests the modification of the immunogenic sequence.

A deregulation of apoptosis, for example in the execution phase, or in the clearance of apoptotic material by scavenger phagocytes (polymorphonuclear neutiophils macrophages), might predispose patients with appropriate genetic background to a break of tolerance toward cell-associated antigens and to the emergence of autoimmunity. An important question is to identify which antigen (s) and which epitope (s) may be relevant in the initiation of the autoimmune process. Proteins bearing these autoepitopes are present in vivo and it is possible to identify them as well as the relevant epitope (s).

Definitions:
■ *modifier peptide* : a chemical modification of one or several amino acid residues constituting the peptide such as, but not limited to, phosphorylation, acetylation, and methylation ; the modification must allow the recognition of the modified peptide as an antigen by antigen-presenting cells (APC's), and, in this context, by auto-reactive CD4+T cells directed toward the naturally occurring, or possibly naturally modified, peptide (if the modification occurs naturally at the particular amino acid position as the peptide exists in the cell).
■ *Altered Peptide Ligands (APL):* peptides recognized as antigens by APCs and, in the context of the present invention, by CD4+T cells, and able to induce a cascade of events different from the naturally occurring one (e. g., stimulation of other cells, production of different cytokines, difference in the signaling, etc. ); this can lead to a significant diminution or even a complete cessation of the deleterious autoimmune response.
■ *Systemic lupus erythematosus (SLE)* : a syndrome of multi- factorial etiology characterized by widespread inflammation in humans; in SLE, the body's natural defenses against infection are turned against the body through the production of antibodies against the body's own cells ; these antibodies fight against the body's blood cells, organs and tissues, causing chronic diseases.
■ *epitope* : that part of an antigen recognized by an antigen receptor.
■ *apoptosis* : form of programmed cell death characterized by endo-nuclear digestion of DNA.

It has been proposed that a pre-existing network linked to epitopes of specific antigens exist and that malfunctioning of this network produces autoimmune disease states. Suggestions have been made that peptide analogs that peptide analogs that will bind to the appropriate MHC molecule and block the response to autoantigens can be utilized to turn off an ongoing autoimmune response. This manner of proceeding has certain disadvantages including impairment of microbial defenses and the requirement for very high doses of the peptide.

### SUMMARY OF THE INVENTION

The present invention, in one embodiment, provides a modified peptide corresponding to SEQ ID NO:1 RIHMVYSKRSGKPRGYAFIEY [SEQ ID NO: 11], wherein
S in position 7 and/or in position 10 is modified by phosphorylation. In another alternative embodiment, the present invention provides a modified peptide as defined above, wherein S in position 7 and/or 10 is modified by phosphorylation, and the acetylation of K in position 8 and/or in position 12 is modified by acetylation. The present invention provides the modified peptide as defined above wherein S in position 10 is modified by phophorylation.

In a second embodiment, the present invention contemplates a pharmaceutical composition in dosage unit form, comprising in combination a pharmaceutically acceptable carrier and an effective amount of at least one modified peptide as defined above. Preferably, the modified peptide is chemically modified RIHMVYSKRSGKPRGYAFIEY [SEQ ID NO: 1]. More preferably, the modified peptide of SEQ ID NO: 1 is chemically modified by any combination of the phosphorylation of S in position 7 and/or in position 10, and the acetylation of K in position 8 and/or in position 12. Alternatively, the composition of the present invention comprises a modified peptide of SEQ ID NO: 1 wherein the peptide is chemically modified by phosphorylation in the 10 position. In one aspect of this embodiment, the present invention provides a composition that is in the form of a lozenge, tablet, gelatin, capsule, drop, pill, or liposome.
Alternatively, the composition is in the form of a solution.

The invention also encompasses the use of a modified peptide as defined above, for the preparation of a medicine for treating autoimmune diseases. In yet another alternative embodiment, the present invention encompasses a method for treating autoimmune diseases, wherein the method comprises the step of administering to a patient in need of such treatment a pharmaceutical composition comprising a modified peptide prepared according to the present invention in an amount sufficient to effect the desired treatment. In addition, the invention also encompasses the use of a modified peptide as defined above, for the preparation of a medicine for treating systemic lupus erythematosus. The present invention contemplates a method of treating systemic lupus erythematosus that comprises administering to a patient in need of such treatment a pharmaceutical composition comprising a peptide modified according to the teachings of the present invention in an amount sufficient to effect said treatment.

The present invention also provides a modified peptide that is the product of the chemical modification by at least one of the methods selected from the group consisting of phosphorylation, acetylation, or methylation of RIHMVYSKRSGKPRGYAFIEY [SEQ ID NO : 1].

### DETAILED DESCRIPTION OF THE INVENTION

In accordance with the practice of the present invention, it has been found that it is possible to create a family of modified peptides, wherein the therapeutically relevant epitopes of the peptides have been modified so as to transform the peptides into "altered peptide ligands" (APL's). These APL's are capable of acting as decoys towards CD4+ T cells, thereby drastically reducing their immune effectiveness and, consequently, the organism's autoimmune response.

The APL's are created by a chemical modification of one or more of the several amino acid residues constituting the peptide including without limitation, and by way of example, phosphorylation, acetylation and methylation.
The modification must allow recognition of the modified peptide as an antigen by antigen presenting cells and in this context by auto-reactive CD4+T cells directed against the naturally occurring, or possibly modified, peptides. The modification serves to convert the peptides into altered peptide ligands that are recognized as antigens by antigen-presenting cells and, in the practice of the instant invention, by CD4 + T cells, functioning to significantly reduce or entirely eliminate a deleterious autoimmune response.

Specifically, the present invention is directed toward a process that comprises modifying a therapeutically relevant epitope of a peptide recognized as an antigen by the CD4+ T cells that are responsible for inducing an autoimmune response. Furthermore, the present invention lies in the synthetic peptides defined as "modified" from their natural epitopes and able to demonstrate an effective activity against autoimmune diseases, and their preparation. In particular, the invention comprises modifications, including the phosphorylation of S in position 7 and/or 10; the acetylation of K in position 8 and/or 12; and any combination thereof, of the specific peptide RIHMVYSKRSGKPRGYAFIEY (SEQ ID NO : 1), which peptide is a 21-amino acid segment of the 70 kDa snRNP protein, corresponding to residues 131 to 151.

The U 1-small nuclear ribonucleoprotein particle (snRNP) belongs to the most complex autoantigens known to be recognized in systemic autoimmune diseases. This particle consists of an RNA backbone and eleven associated proteins that are immunogenic in patients suffering from systemic lupus erythematosus and mixed connective tissue disease (MCTD). Antibodies directed against the 70 kDa protein (also called U1 70 kDa or RNP-68) are specific for Sharp's syndrome. Up to 100% of all patients suffering from mixed connective tissue disease exhibit these autoantibodies. Apart from the 70 kDa protein and the Sm proteins, the U1-snRNP-complex also contains the proteins A and C. Patients suffering from SLE and MCTD exhibit antibodies directed against the proteins A, C and 70 kDa. Autoantibodies against Sm proteins are of pathognomonic importance for diagnosing SLE. A patient with a positive anti- Sm finding is confronted with the diagnosis of SLE. But a negative finding does not exclude SLE. Anti-Sm is detected in 10% of Caucasian and 30% of Black and Chinese patients with SLE. The detection of autoantibodies against Sm belongs to the criteria for the diagnosis of SLE of the American College of Rheumatology (ACR) published in 1982.

Furthermore, the advantageous properties of the substances of the invention are accompanied by low toxicity.

These substances are particularly suited to the development of pharmaceutical compositions.

Other characteristics and advantages of the invention will become apparent from the example which follows relating to the preparation of the modified peptide and to the study of its activity against an autoimmune disease.
Systemic lupus presents an easy clinical symptom to follow because of the large urinary protein excretion that accompanies the disorder that results from an associated renal condition, glomerulonephritis. Levels of protein secretion were followed to assess the effectiveness of the modified peptide in addressing the underlying autoimmune disorder.

### Example 1

The specific peptide RIHMVYSKRSGKPRGYAFIEY (SEQ ID NO : 1), which is a 21-amino acid sequence of the 70 kDa snRNP protein corresponding to residues 131-151, was synthesized in its phosphorylated form (in the 10 position-P10). This self-protein, which is normally present in each cell, is recognized by T-cells and antibodies from lupus patients and mice as an antigen toward which the immune system is reacting, generating a so-called autoimmune reaction. After multiple administrations (similar to a vaccine) in mice, the modified peptide induced a significant improvement in the survival rate (55% compared to 0%), whereas administration of the natural (non-phosphorylated) peptide, and the peptide phosphorylated in position 7, were unable to improve significantly the survival rate. Preliminary data of the acetylated peptide demonstrated an even stronger activity. Furthermore, the modified peptide P10 also increased significantly the production of IL-2 (interluekin-2) by CD4+ T cells and reduced the urine protein excretion in treated mice.

The pharmaceutical compositions of the invention contain an efficacious amount of a modified peptide in combination with an inert pharmaceutical vehicle.

In view of their ability to block the response to autoantigens, the compositions can be used in the treatment of those diseases associated with autoimmunity, including rheumatoid arthritis, Addison's disease, scleroderma, systemic lupus erythematosus, myasthenia gravis, juvenile diabetes, and the like.

The pharmaceutical compositions of the invention may be administered with different forms and by different routes, including nasal, rectal, oral and by injection. In the case of administration by the oral route, recourse may be had, in particular, to tablets, pills, lozenges, gelatin capsules, drops and even liposome. Other forms of administration comprise sterile or sterilizable solutions which can be injected by the intravenous, subcutaneous or intramuscular route.

In the case of the liposome form of administration, this method of presentation can be utilized to advantage by encapsulating the modified peptide in acid-resistant liposomes so that it can only enter the MHC class 1 route and stimulate CD8+ T cells. Antigens within acid-sensitive liposomes become associated with both class I and class 11 molecules and evidence high response efficiency. It should therefore be possible to use a single-shot liposome vaccine with multiple potentialities which incorporate several modified peptides, i.e., antigens, different adjuvants and specialized targeted molecules for accomplishing the desired treatment.

For practical and economic reasons, whatever route is selected, the minimum number of doses and the least amount of modified peptide should be involved.

## Claims

1. A modified peptide in which at least one of the amino acid residues constituting the peptide has been chemically modified.

2. A modified peptide according to claim 1, wherein said chemical modification is carried out by one or more methods selected from the group consisting of phosphorylation, acetylation and methylation.

3. A modified peptide according to claim 1, wherein said peptide is RIHMVYSKRSGKPRGYAFIEY [SEQ ID NO: 1].

4. A modified peptide according to claim 3, wherein said chemical modification is carried out by phosphorylation of S in position 7 and/or position 10.

5. A modified peptide according to claim 3, wherein said chemical modification is carried out by acetylation of K in position 8 and/or in position 12.

6. A modified peptide according to claim 3, wherein said chemical modification is carried out by any combination of the phosphorylation of S in position 7 and/or 10, and the acetylation of K in position 8 and/or 12.

7. A modified peptide according to claim 1, which peptide is derived from a 70 kDa snRNP protein.

8. A modified peptide according to claim 3, which peptide has been phosphorylated in the 10 position.

9. A pharmaceutical composition in unit dosage form, comprising in combination a pharmaceutical acceptable carrier and at least one modified peptide according to claim 1, in an amount effective for reducing or eliminating a deleterious autoimmune response.

10. The composition of claim 9 wherein said peptide is chemically modified RIHMVYSKRSGKPRGYAFIEY [SEQ ID NO: 1].

11. The composition of claim 10, wherein said peptide is chemically modified by any combination of the phosphorylation of S in position 7 and/or in position 10 and the acetylation of K in position 8 and/or in position 12.

12. The composition of claim 10, wherein said peptide is chemically modified by phosphorylation in the 10 position.

13. The composition according to claim 9 which is in the form of a lozenge, tablet, gelatin, capsule, drop, pill, or liposome.

14. The composition according to claim 9 which is in the form of a solution.

15. The pharmaceutical composition according to claim 9 for its use in the treatment of an autoimmune disease.

16. The pharmaceutical composition according to claim 9 wherein the A method of autoimmune disease is systemic lupus erythematosus.
